# EUROPÄISCHE PATENTANMELDUNG

(11) **EP 3 549 557 A1**
(43) Veröffentlichungstag der Anmeldung: **09.10.2019**
(21) Anmeldenummer: 18165617.4
(22) Anmeldetag: 04.04.2018
(51) Int. Cl.: A61F 2/966

(54) **STRUKTUR FÜR EINE KATHETERHÜLSE UND KATHETERHÜLSE**

(71) Anmelder: Biotronik AG, 8180 Bülach (CH)
(72) Erfinder: Fargahi, Amir, 8180 Bülach (CH)
(74) Vertreter: Keck, Hans-Georg

(57) **Zusammenfassung**

Die Erfindung betrifft eine röhrenförmige Struktur für eine Katheterhülse (4), welche sich durch eine besonders hohe Biegeflexibilität auszeichnet. Die röhrenförmige Struktur weist mindestens ein S-förmiges Profilband (50, 150, 250, 350, 450) auf, das entlang der Struktur in einer Helix angeordnet ist, wobei das mindestens eine S-förmige Profilband (50, 150, 250, 350, 450) im Bereich jeder Seitenkante jeweils einen U-förmigen Längsabschnitt (51, 52, 151, 152, 251, 252, 351, 352, 451, 452) aufweist, wobei jeweils ein U-förmiger Längsabschnitt (51, 52, 151, 152, 251, 252, 351, 352, 451, 452) des Profilbands mit dem gegenüber liegenden, U-förmigen Längsabschnitt (51, 52, 151, 152, 251, 252, 351, 352, 451, 452) eines unmittelbar benachbarten Gangs der Helix desselben Profilbands (50, 150, 250, 350, 450) oder eines weiteren, in der Helix benachbart angeordneten S-förmigen Profilbands (50, 150, 250, 350, 450) ineinander greift. Die Erfindung betrifft ferner eine entsprechende Katheterhülse (4) sowie einen entsprechenden Katheter.

## Beschreibung

Die vorliegende Anmeldung betrifft eine röhrenförmige Struktur für eine Katheterhülse sowie eine entsprechende Katheterhülse und einen entsprechenden Katheter.

Medizinische Implantate, insbesondere intraluminale Endoprothesen, für unterschiedlichste Anwendungen sind in großer Vielfalt aus dem Stand der Technik bekannt. Implantate sind z. B. endovaskuläre Prothesen oder sonstige Endoprothesen, beispielsweise Stents (Gefäß-Stents (vaskuläre Stents, einschließlich Stents für die Anwendung im Bereich des Herzens und Herzklappen-Stents, zum Beispiel Mitralklappen-Stents, Pulmonalklappen-Stents) und Gallengang-Stents), Endoprothesen zum Verschließen von persistierenden Foramen Ovale (PFO), Stentgrafts zur Behandlung von Aneurysmen, Endoprothesen zum Verschließen eines ASG (Vorhofscheidewanddefekt, Atrioseptaldefekt) sowie Prothesen im Bereich des Hart- und Weichgewebes.

Ein derartiges Implantat nimmt üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels eines Katheters in das zu behandelnde Gefäß oder Organ durch enge Gefäße hindurch eingeführt und an der zu behandelnden Stelle positioniert werden. Die Fixierung im komprimierten Zustand erfolgt hierbei häufig mittels einer am distalen Ende des Katheters angeordneten Katheterhülse (auch als Kapsel bezeichnet). In dem expandierten Zustand verbleibt das Implantat in dem Gefäß oder Organ und ist dort festgelegt, nachdem der Katheter aus dem Körper des Behandelten entfernt wurde. Beispielsweise wird bei einer Transkatheter-Aortenklappenimplantation (endovaskulärer Aortenklappenersatz, englisch transcatheter aortic valve implantation, TAVI) eine künstliche Aortenklappe in einem röhrenförmigen Gerüst in das Herz eingebracht, für dessen Implantation heutzutage vorwiegend Katheter aus Kunststoffen beziehungsweise Komposits eingesetzt werden, deren Biegsamkeit und Flexibilität begrenzt sind. Mittels Katheter wird die Klappe in Position gebracht. Anschließend wird sie entfaltet und verankert. Die körpereigene Aortenklappe wird dabei nicht entfernt, sondern durch das Implantat verdrängt. Bei einem selbstexpandierenden Implantat aus einer Formgedächtnislegierung geht das Implantat bei Überschreiten einer Umwandlungstemperatur oder bei Wegfall einer auf das Implantat ausgeübten mechanischen Spannung in den expandierten Zustand über.

Während des Einbringens des Implantats mittels des Katheters wird die Katheterhülse entlang von Blutgefäßen geführt beziehungsweise positioniert. Hierbei passt sich die Katheterhülse der jeweiligen Form des inneren Volumens des Gefäßes an und wird hierdurch einer wechselnden Biegekraft beziehungsweise einer wechselnden Druck- und Zugbelastung ausgesetzt. Insbesondere der Durchgang eines Katheters durch den Aortenbogen führt zu einer starken Verformung der Katheterhülse. Die Verformung der Katheterhülse kann die Funktionsweise des Katheters und/oder des in der Katheterhülse angeordneten Implantats beeinträchtigen.

Beispiele für Katheterhülsen nach dem Stand der Technik können den Druckschriften WO 2011/133368 A1, EP 1 723 937 A1 und US 2011/0098804 A1 entnommen werden. Die Druckschrift US 2011/0098804 A1 offenbart eine Katheterhülse, welche im mittleren Abschnitt eine Vielzahl von helixartig geformten Ringen aufweist, die mittels entsprechenden Einschnitten voneinander getrennt sind. Die Einschnitte erstrecken sich in Umfangsrichtung über weniger als 180°, so dass die Ringe mittels einer oder mehrerer in Längsrichtung verlaufenden Rippen verbunden sind. Eine ähnliche Form einer Katheterhülse (Katheterschlauch) wird auch in der Druckschrift EP 1 723 937 A1 offenbart. Die Druckschrift WO 2011/133368 A1 zeigt eine Katheterhülse mit in Längsrichtung verlaufenden Schlitzen.

Die Aufgabe der vorliegenden Erfindung besteht somit darin, eine Struktur für eine Katheterhülse anzugeben, welche während der Implantation eine höhere Biegeflexibilität und/oder Druck- beziehungsweise Zugbelastbarkeit besitzt. Entsprechend besteht die Aufgabe darin, die Katheterhülse eines Katheters mit einer höheren Biegeflexibilität auszustatten.

Die obige Aufgabe wird gelöst durch die Struktur mit den Merkmalen des Anspruchs 1.

Die erfindungsgemäße röhrenförmige (hohlzylindrische) oder hülsenförmige Struktur weist insbesondere mindestens ein S-förmiges Profilband auf, das entlang der Struktur in einer Helix angeordnet ist, wobei das mindestens eine S-förmige Profilband im Bereich jeder Seitenkante jeweils einen U-förmigen Längsabschnitt aufweist, wobei jeweils ein U-förmiger Längsabschnitt des Profilbands mit dem gegenüber liegenden, U-förmigen Längsabschnitt eines unmittelbar benachbarten Gangs der Helix desselben Profilbands oder eines weiteren, in der Helix benachbarten S-förmigen Profilbands ineinander greift. In dem zweiten Fall weist die Struktur somit mindestens zwei ineinander greifende und jeweils in einer Helix verlaufende Profilbänder auf. Die mindestens zwei Profilbänder bilden eine mehrgängige Helix. In dem ersten Fall wird die Helix lediglich von einem einzigen Profilband gebildet.

Die rohrförmige Struktur bildet dabei vorzugsweise einen Innendurchmesser im Bereich zwischen 3,7 mm und 5,7 mm, bevorzugt zwischen 4,4 mm und 5 mm aus. Der Außendurchmesser ist vorzugsweise 6 mm oder kleiner, bevorzugt 5,3 mm oder kleiner, besonders bevorzugt 4,7 mm oder kleiner und insbesondere ist der Außendurchmesser bevorzugt 4 mm oder kleiner. Es ist dem Fachmann jedoch klar, dass der Innendurchmesser in jedem Fall kleiner als der Außendurchmesser ist.

Das S-förmige Profilband oder die Profilbänder ist/sind derart entlang einer Schraubenlinie oder einer Wendel oder Spirale (Helix) angeordnet bzw. gewunden, dass es/sie sich mit konstanter Steigung um den Mantel der röhrenförmigen Struktur entlang erstreckt/erstrecken. Hierbei überwindet die jeweilige Helix innerhalb einer Windung über 360° die sogenannte Ganghöhe, die parallel zur Längsachse der Helix verläuft. Das Profilband oder die Profilbänder, wie unten noch ausführlicher beschrieben wird/werden, greift/greifen ineinander, so dass eine vollständige Röhre oder Hohlzylinder - die erfindungsgemäße Struktur - ausgebildet wird, die keine Freiräume (z. B. Schlitze) zwischen den Profilbändern aufweist. Die Hohlzylinderform wird durch das Profilband oder die Profilbänder, die in der Helix angeordnet sind, vollständig ausgefüllt beziehungsweise abgedeckt. Bei der Verwendung mehrerer Profilbänder sind diese entlang des Umfangs der Helix nebeneinander angeordnet. Die durch das Profilband oder die Profilbänder beschriebene Helix kann rechtsgängig (d. h. im Uhrzeigersinn gewunden) oder linksgängig (d. h. entgegen dem Uhrzeigersinn gewunden) ausgebildet sein.

Jedes Profilband (auch als profiliertes Flachband bezeichnet) besitzt einen S-förmigen Querschnitt. Der S-förmige Querschnitt setzt sich zusammen aus zwei U-förmigen Längsabschnitten, wobei ein erster U-förmiger Längsabschnitt distal und ein zweiter U-förmiger Längsabschnitt proximal in Bezug auf die Struktur angeordnet sind, sowie einem zwischen den Längsabschnitten liegenden, d. h. mittigen, gerade verlaufenden Abschnitt. Die beiden U-förmigen Abschnitte, die jeweils den Längsabschnitt ausbilden, sind in Bezug auf die radiale Richtung der röhrenförmigen Struktur in verschiedene Richtungen gebogen. Ein erster U-förmiger Längsabschnitt ist nach innen gebogen, während ein zweiter U-förmiger Längsabschnitt nach außen gebogen ist. Der mittige gerade Abschnitt verbindet die beiden U-förmigen Längsabschnitte miteinander über einen mehr oder weniger runden Übergang. Um eine Beweglichkeit der ineinander greifenden Profilbänder zueinander zu gewährleisten, sind die beiden U-förmigen Längsabschnitte derart gestaltet, dass jeweils ein Schenkel des "U" länger ist als der andere Schenkel. Insbesondere ist der Schenkel, der mit dem jeweils anderen Längsabschnitt über den mittigen Abschnitt verbunden ist, länger als der andere Schenkel.

Durch das Ineinandergreifen der S-förmigen Profilbänder insbesondere in ihren jeweiligen U-förmigen Längsabschnitten können sich die Profilbänder zueinander verdrehen und verschieben, ohne dass eine signifikante Verformung der Profilbänder erfolgt. Folglich besitzt die erfindungsgemäße Struktur eine hohe Biegeflexibilität.

In einem Ausführungsbeispiel verläuft der mittige Abschnitt eines S-förmigen Profilbands in radialer Richtung der Struktur. Alternativ verläuft der mittige Abschnitt eines S-förmigen Profilbands schräg zu der radialen Richtung der Struktur. Hierdurch kann das Profil beziehungsweise seine Biegeflexibilität an die jeweiligen Gegebenheiten bei der Implantation, d. h. dem Aufbau und der Form der Gefäße, durch die das Implantat verschoben werden soll, angepasst werden.

Das mindestens eine, die erfindungsgemäße Struktur ausbildende Profilband weist vorzugsweise mindestens ein metallisches Material der Gruppe umfassend Stahl, Co-Cr-Legierungen, Nitinol und Kupfer-Legierungen und/oder ein steifes Polymermaterial auf.

Die obige Aufgabe wird ferner durch eine Katheterhülse gelöst, welche sich insbesondere für das Einbringen eines stentgestützten Herzklappenimplantats eignet, wobei dessen Stentgerüst vorzugsweise selbstexpandierend ausgebildet ist. Die erfindungsgemäße Katheterhülse weist eine Versteifungshülse und eine erste Polymerlage auf, die in radialer Richtung innerhalb (d. h. in) der Versteifungshülse angeordnet ist. Ferner ist eine zweite Polymerlage vorgesehen, die in radialer Richtung außerhalb (d. h. außen auf) der Versteifungshülse angeordnet ist, wobei die obige erfindungsgemäße Struktur vorzugsweise einen mittigen Abschnitt der Versteifungshülse und/oder einen proximalen Abschnitt der Versteifungshülse ausbildet. Die oben beschriebene, erfindungsgemäße Struktur ist daher entsprechend innerhalb der beiden Polymerlagen angeordnet. In einem bevorzugten Ausführungsbeispiel ist die Katheterhülse über einen hülsenförmigen Konnektor mit dem Außenschaft eines Katheters verbunden. An seinem distalen Ende kann die Katheterhülse eine sogenannte Krone aus einem röhrenförmigen Strutgeflecht aufweisen, welche sich beim Freisetzen des Implantats besonders flexibel in radialer Richtung aufweitet und gegebenenfalls auch wieder zusammenfaltet.

In einer alternativen Ausgestaltung der Erfindung erstreckt sich die erfindungsgemäße Struktur über die gesamte Länge der Katheterhülse.

Die erfindungsgemäße Katheterhülse, auch als Implant Capsule bezeichnet, besitzt aufgrund der erfindungsgemäßen Struktur, die die Versteifungshülse ausbildet, eine höhere Biegeflexibilität und kann sich daher komplizierten Gefäßformen besser anpassen. Durch die Verschieblichkeit der Windungen der röhrenförmigen Struktur in Längsrichtung zueinander wird außerdem eine hohe Zug- und Druckbelastbarkeit erzielt. Gleichzeitig wird durch die Polymerlagen ein Schutz der Versteifungshülse realisiert. Zudem wird eine gute Beweglichkeit des Implantats relativ zu der Katheterhülse erreicht.

Vorzugsweise weist die erste Polymerlage mindestens ein Material der Gruppe umfassend Teflon und HDPE und die zweite Polymerlage mindestens ein Material der Gruppe umfassend PEBAX auf. Ferner kann für die erste und die zweite Polymerlage mindestens ein Material der Gruppe umfassend Polyolefine, Polyamide, Polyurethane und Polyharnstoffe, Polyether, Polyester, Polyoxiden, Polysulfiden (PPS), Parax, Polyetheretherketone (PEEK) und deren Copolymere, fluorierten Polymere, die zuvor genannten Polymere im Gemisch mit Bariunsulfat-Pulver und/oder Wolfram-Pulver eingesetzt werden.

Die obige Aufgabe wird analog durch einen Katheter mit der oben beschriebenen Katheterhülse gelöst, wobei die Katheterhülse zur Aufnahme eines zusammengefalteten Implantats dient und ausgebildet ist sowie mit dem Außenschaft des Katheters verbunden ist. Das Implantat ist vorzugsweise über einen sogenannten Prothesenkonnektor auf dem Innenschaft des Katheters fixiert. Wie bei herkömmlichen Kathetern ist der Außenschaft auf dem Innenschaft geführt und beweglich.

Die erfindungsgemäße Katheterhülse ist vorzugsweise Teil eines Katheters zum Einbringen eines stentgestützten Herzklappenimplantats. Ein derartiges Herzklappenimplantat besteht aus einer Klappenanordnung, bevorzugt aus Perikard, welche an einem Stentgerüst befestigt ist und von diesem getragen wird. Das Stentgerüst kann ballonexpandierbar oder bevorzugt selbstexpandierend sein. Ein selbstexpandierendes Stentgerüst wird dabei bevorzugt aus einem Formgedächtnismaterial insbesondere Nitinol ausgeführt. Ein derartiger Katheter zum Einbringen eines Herzklappenimplantats umfasst mindestens zwei Katheterschäfte, einen Innenschaft und einen den Innenschaft umgebenden Außenschaft. Der Innenschaft weist ein Lumen für einen Führungsdraht auf, das Herzklappenimplantat ist auf dem distalen Bereich des Innenschaftes angeordnet. Distal wird im Rahmen dieser Anmeldung als vom Behandler entfernt verstanden. Der Behandler hat entsprechend das proximale Ende des Katheters in der Hand, während sich das distale Ende bei Implantation des Herzklappenimplantats im Körper befindet. Der Außenschaft ist gegenüber dem Innenschaft axial verschieblich. Der distale Bereich des Außenschafts, der das Herzklappenimplantat umgibt, ist als erfindungsgemäße Katheterhülse ausgestaltet. Im Falle eines selbstexpandierenden Herzklappenimplantats hält die Katheterhülse das Herzklappenimplantat in seiner komprimierten Form. Durch eine axiale Verschiebung des Außenschaftes gegenüber dem Innenschaft wird das selbstexpandierende Herzklappenimplantat zumindest teilweise nicht mehr von der Katheterhülse überdeckt und expandiert. Wenn kein Teil des Herzklappenimplantats mehr von der Katheterhülse überdeckt ist, ist das Herzklappenimplantat vollständig expandiert und freigesetzt.

In dieser Ausgestaltung kommen die Vorteile der Erfindung insbesondere zum Tragen. Die erfindungsgemäße rohrförmige Struktur in der erfindungsgemäßen Katheterhülse ermöglicht ein sicheres Wiedereinfangen des Herzklappenimplantats, wenn dieses teilweise freigesetzt ist. Wenn der Behandler bei einem teilweise freigesetzten Implantat eine Fehlfunktion oder nicht optimale Positionierung feststellt, besteht der Bedarf dieses teilweise freigesetzte Implantat wieder zu komprimieren und erneut zu positionieren oder aus dem Körper zu entfernen. Dazu muss die Katheterhülse wieder vollständig über das teilweise freigesetzte Implantat geschoben werden, um dieses in seine komprimierte Form zu überführen. Die erfindungsgemäße rohrförmige Struktur als Teil der erfindungsgemäßen Katheterhülse stellt eine gute axiale Steifigkeit und hohe Radialkraft zur Verfügung ohne gleichzeitig die Biegeweichheit/Biegefähigkeit zu verlieren.

Bevorzugt ist die Versteifungshülse über einem metallischen Verbinder mit dem Außenschaft 3 verbunden. Ein derartiger Verbinder sorgt für einen guten Übergang und eine gute Kraftübertragung von Außenschaft auf Katheterhülse.

Zweckmäßigerweise geht die erste Polymerlage 41, bevorzugt nahtlos, in eine innere Polymerlage des Außenschaftes 3 und die zweite Polymerlage 42, bevorzugt nahtlos, in eine äußere Polymerlage des Außenschaftes 3 über, wobei besonders bevorzugt der Außenschaft zwischen innerer und äußerer Polymerlage eine metallische Verstärkung aufweist. Häufig muss auch der Außenschaft verstärkt werden. In dieser Ausgestaltung der Erfindung kann die gleiche innere Polymerlage und die gleiche äußere Polymerlage für Katheterhülse und Außenschaft verwendet werden. Insbesondere können diese als ein Werkstück extrudiert werden, wenn der Durchmesser der Katheterhülse und des Außenschafts gleich sind. Die zweite Polymerlage 42 kann auch als Schrumpfschlauch aufgebracht werden, unabhängig davon, ob die Katheterhülse und der Außenschaft einen identischen Außendurchmesser aufweisen.

Weitere Ziele, Merkmale, Vorteile und Anwendungsmöglichkeiten der Erfindung ergeben sich auch aus der nachfolgenden Beschreibung von Ausführungsbeispielen der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

### Es zeigen schematisch

- Fig. 1: einen erfindungsgemäßen Katheter vor der Implantation eines Implantats in einer perspektivischen Ansicht von der Seite,
- Fig. 2: einen distalen Abschnitt des Katheters aus Fig. 1 in einer perspektivischen Ansicht von der Seite nach dem Freisetzen des Implantats,
- Fig. 3: eine erfindungsgemäße Katheterhülse mit Außenschaft in einer perspektivischen Ansicht von der Seite,
- Fig. 4: einen Querschnitt durch die Katheterhülse gemäß Fig. 3 an der Stelle C (siehe Fig. 3),
- Fig. 5: ein erstes Ausführungsbeispiel eines Profilbands einer erfindungsgemäßen Struktur in einer perspektivischen Ansicht von der Seite,
- Fig. 6: ein zweites Ausführungsbeispiel eines Profilbands einer erfindungsgemäßen Struktur in einem Querschnitt,
- Fig. 7: einen Längsschnitt durch die Wand einer erfindungsgemäßen Katheterhülse mit zwei ineinander greifenden Gängen eines Profilbands gemäß Fig. 5, abgerollt in eine Ebene
- Fig. 8: einen Längsschnitt durch zwei ineinander greifende Gänge der Helix eines Profilbands gemäß Fig. 6,
- Fig. 9: einen Längsschnitt durch zwei ineinander greifende Gänge der Helix eines dritten Ausführungsbeispiels eines Profilbands einer erfindungsgemäßen Struktur,
- Fig. 10: einen Längsschnitt durch zwei ineinander greifende Gänge der Helix eines vierten Ausführungsbeispiels eines Profilbands einer erfindungsgemäßen Struktur,
- Fig. 11: einen Gang der Helix des Profilbands gemäß Fig. 10 in einer perspektivischen Ansicht von der Seite,
- Fig. 12: einen Längsschnitt durch zwei ineinander greifende Gänge der Helix eines fünften Ausführungsbeispiels eines Profilbands einer erfindungsgemäßen Struktur,
- Fig. 13: eine Versteifungshülse einer erfindungsgemäßen Katheterhülse in einer perspektivischen Ansicht von der Seite mit einer Struktur gemäß Fig. 12 in gekrümmtem Zustand,
- Fig. 14: einen Abschnitt des Profilbands gemäß Fig. 12, abgerollt in eine Ebene und in einer Ansicht von oben,
- Fig. 15: einen Gang der Helix des Profilbands gemäß Fig. 12 in einer perspektivischen Ansicht von der Seite,
- Fig. 16: eine Krone mit einem Haken zur Befestigung der Krone an der Katheterhülse und
- Fig. 17: ein Ausführungsbeispiel des Profilbands einer erfindungsgemäßen Struktur mit einem Schlitz zur Aufnahme des Hakens einer Krone in einem Querschnitt.

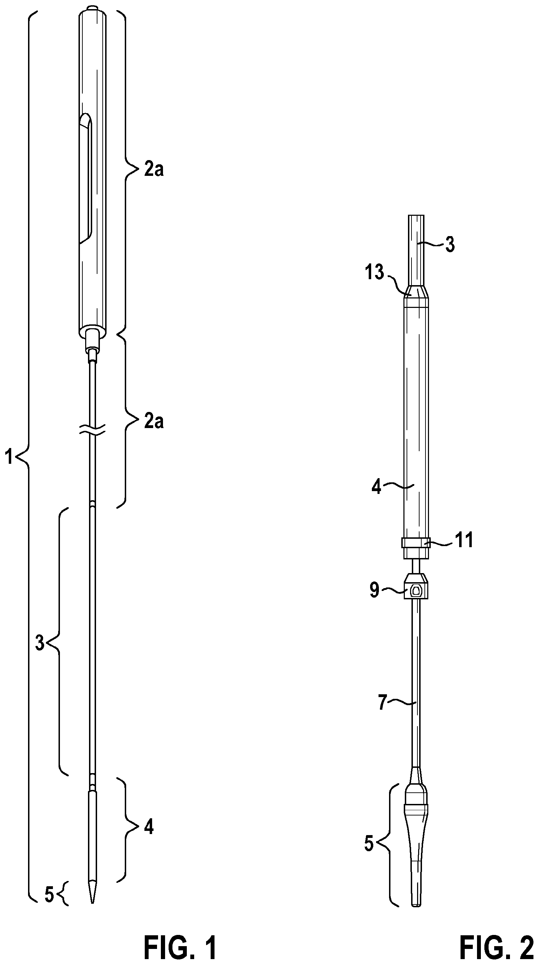

Fig. 1 zeigt einen erfindungsgemäßen Katheter 1 mit einem am proximalen Ende des Katheters angeordneten Handgriff 2a, einen Stabilisierabschnitt 2b, einen Außenschaft 3 sowie einer auf dem Außenschaft 3 angeordneten oder mit dem Außenschaft 3 verbundenen Katheterhülse 4. Am äußersten distalen Ende ist eine stumpfe Katheterspitze 5 vorgesehen. Der Stabilisierabschnitt 2 schirmt den zurückziehbaren Außenschaft 3 gegen das Einführelement (Introducer) und die Gefäßwand ab, sodass der Außenschaft 3 frei zurückgezogen werden kann. Der Handgriff 2a dient dem Beladen, Freisetzen und Zurückziehen eines in der Katheterhülse 4 angeordneten Implantats, beispielsweise einer stentbasierten Herzklappenprothese. Eine derartige stentbasierte Herzklappenprothese besteht im Wesentlichen aus einem selbstexpandierenden Stentgerüst, welches das Grundgerüst des Implantats bildet, und einer darin befestigten Klappenanordnung, beispielsweise aus biologischen Gewebe wie Perikardgewebe. Die Katheterspitze 5 bildet das distale Ende eines innerhalb des Außenschafts 3 angeordneten Innenschafts 7 (siehe Fig. 2), wobei die Katheterspitze 5 beispielsweise aus PEBAX im Gemisch mit Bariunsulfat-Pulver bzw. Wolfram-Pulver gefertigt und unter Röntgenbestrahlung sichtbar ist.

Fig. 2 stellt das distale Ende des in Fig. 1 skizzierten Systems nach dem Freisetzen des Implantats dar. In dieser Figur ist auch zu sehen, dass auf dem Innenschaft 7 ein Prothesenkonnektor 9 angeordnet ist, mit dem das Implantat auf dem Innenschaft 7 befestigt ist. Die Katheterhülse 4 kann an ihrem distalen Ende zur Erleichterung der Beobachtung einen unter Röntgenbestrahlung sichtbaren Ring 11 aufweisen. Die Katheterhülse 4 ist beispielsweise mittels eines proximalen Konnektors 13 mit dem Außenschaft 3 verbunden.

Wie bereits oben erläutert wurde, ist das Implantat zunächst in dem in Fig. 1 gezeigten Zustand in der Katheterhülse 4 (auch als Kapsel bezeichnet) im komprimierten Zustand angeordnet und wird durch die Katheterhülse 4 in diesem Zustand gehalten. Die Katheterhülse 4 ist über den Außenschaft 3 mit dem Handgriff 2a verbunden. In diesem Zustand erfolgt der Transport des in der Katheterhülse 4 fixierten und komprimierten Implantats durch die Gefäße des Patienten an den Ort der Behandlung.

Zum Freisetzen des Implantats wird die Katheterhülse 4 nach proximal zurückgezogen. Der Rückzug wird über den Handgriff 2a ausgelöst und über den Außenschaft 3 auf die Katheterhülse 4 übertragen. Hierbei wird zunächst lediglich ein kurzer distaler Abschnitt des Implantats freigesetzt und der Sitz geprüft. Bei ungünstiger Positionierung kann die Katheterhülse mittels des Handgriffs 2a wieder nach distal zurückgeschoben werden, sodass das Implantat wieder durch die Katheterhülse 4 bedeckt und vollständig in den komprimierten Zustand übergegangen ist. Nun kann der Katheter 1 repositioniert werden. Danach kann das Freisetzen des in der Katheterhülse 4 angeordneten Implantats durch Zurückziehen des Außenschafts 3 von Neuem beginnen.

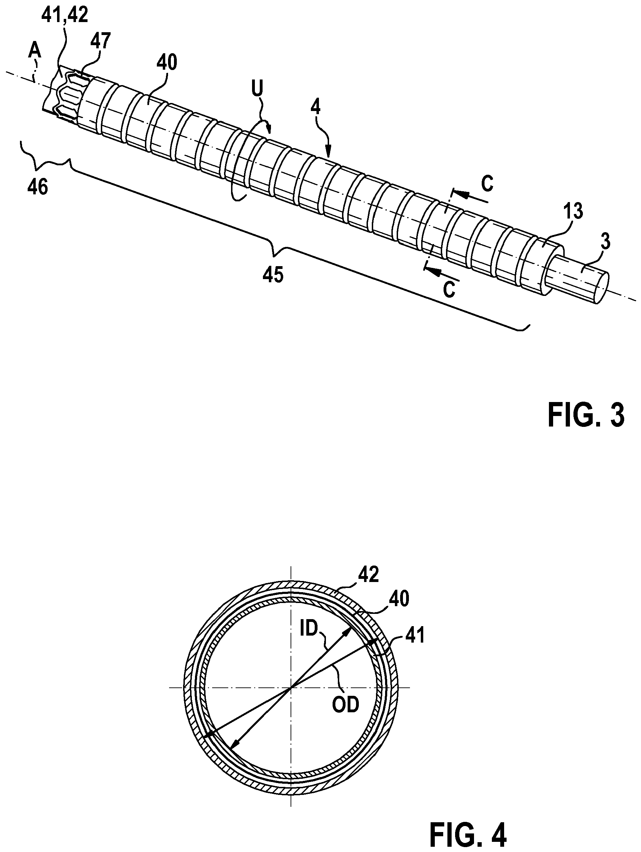

Das in den Fig. 3 und 4 gezeigte Ausführungsbeispiel einer Katheterhülse 4 setzt sich zusammen aus einer Versteifungshülse 40, die zwischen einer innenliegenden ersten Polymerlage 41 und einer außen die Versteifungshülse 40 umgebenden zweiten Polymerlage 42 eingebettet ist. Die Polymerlagen 41, 42 ummanteln die Versteifungshülse 40 (mit anderen Worten: die Polymerlagen bilden ein Jacket aus) und stehen an deren distalen Ende über das distale Ende der Versteifungshülse 40 über. An den überstehenden Bereichen werden die Polymerlagen 41 und 42 miteinander verbunden, beispielsweise verschweißt oder miteinander verklebt.

Die außen liegende zweite Polymerlage 42 der Versteifungshülse 40 kann beispielsweise aus PEBAX 7033 gefertigt sein und eine Dicke von 0,04 mm aufweisen. Die innen liegende erste Polymerlage 41 ist vorzugsweise aus einem reibungsarmen Material gefertigt, beispielsweise aus Teflon oder HDPE. Die erste Polymerlage 41 kann beispielsweise eine Dicke von 0,02 mm aufweisen. Der Außendurchmesser OD der Katheterhülse 4 im Bereich des proximalen Abschnitts 45 beträgt beispielsweise 5 mm bis 7 mm, vorzugsweise 5,8 mm bis 6,2 mm, während der Innendurchmesser ID beispielsweise 5 mm bis 6 mm, vorzugsweise 5,4 mm bis 5,6 mm beträgt. In jedem Fall ist der Innendurchmesser ID kleiner als der Außendurchmesser OD.

Die Versteifungshülse 40 lässt sich unterteilen in einen proximalen Abschnitt 45 und einen distalen Abschnitt 46, wobei der distale Abschnitt auch als Krone 46 bezeichnet wird. In einem alternativen Ausführungsbeispiel der Versteifungshülse weist diese lediglich den proximalen Abschnitt 45 ohne Krone 46 auf. In dieser alternativen Ausgestaltung deckt der proximale Abschnitt 45 die gesamte Länge des Herzklappenimplantats ab. Der proximale Abschnitt 45 und der distale Abschnitt 46 sind hintereinander entlang der Längsachse/Längsrichtung A angeordnet.

Die Versteifungshülse 40 weist vorzugsweise mindestens ein metallisches Material der Gruppe umfassend Stahl, Co-Cr-Legierung, Nitinol, Kupfer-Legierung und/oder ein steifes Polymermaterial auf. An dem äußersten proximalen Ende des proximalen Abschnitts 45 ist die Versteifungshülse 40 über den proximalen Konnektor 13 mit dem Außenschaft 3 verbunden. Die Mittelachse der Versteifungshülse 40 bildet die Längsrichtung A (siehe Fig. 3) der Versteifungshülse 40 beziehungsweise der Katheterhülse 4.

Der distale Abschnitt (die Krone) 46 wird beispielsweise aus einem Strut- oder Drahtgeflecht gebildet, das sich beim Freisetzen des Implantats trichterförmig aufweitet, um das Freisetzen des Implantats (beispielsweise des stentbasierten Herzklappenimplantats) zu erleichtern. Das Geflecht kann beispielsweise mittels Verschweißen mit dem proximalen Abschnitt 45 der Versteifungshülse 40 verbunden sein.

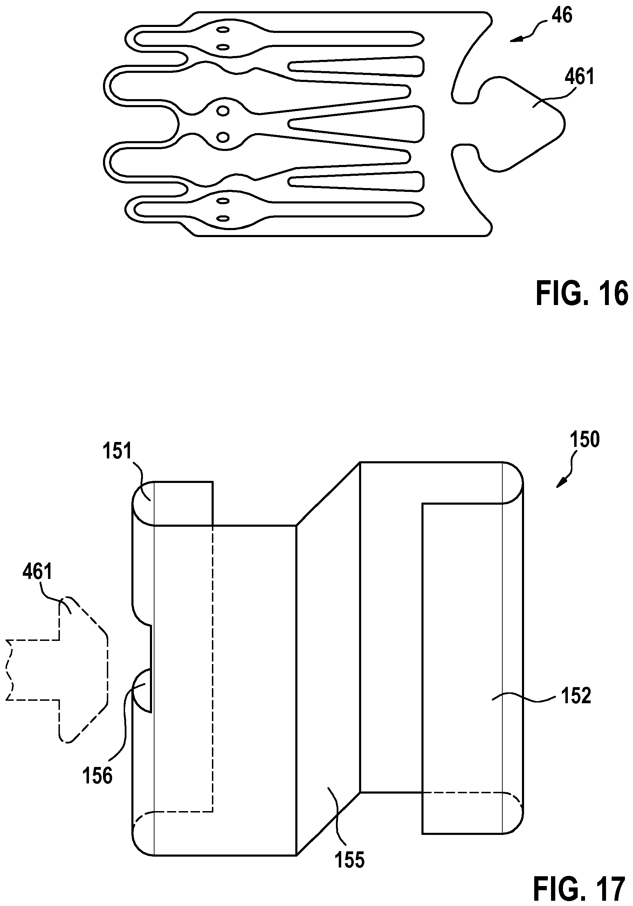

Bevorzugt ist die Versteifungshülse 40 mit einer distalen Krone 46 verbunden, wobei die Verbindung bevorzugt über mindestens ein U-Profil erfolgt, welches in das Profil der rohrförmigen Struktur greift. In dieser Ausgestaltung der Erfindung wird die schon vorhandene Struktur der Versteifungshülse in der Katheterhülse genutzt, um die Krone 46 anzubinden. Als Alternative ist/sind im Profil der Versteifungshülse 40 ein Schlitz 156/mehrere Schlitze 156 vorgesehen, in denen sich ein/mehrere Haken 461 der Krone 46 befestigen lässt/lassen (siehe Fig. 16 und 17). Eine Krone 46 hat den Vorteil, dass sie leichter über ein teilweise freigesetztes Implantat schieben kann und dadurch das Resheathing des Implantats erleichtert.

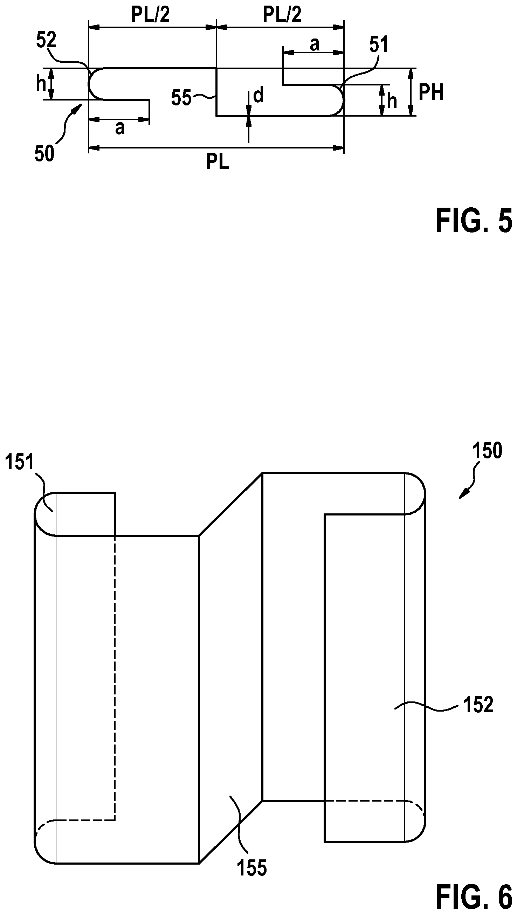

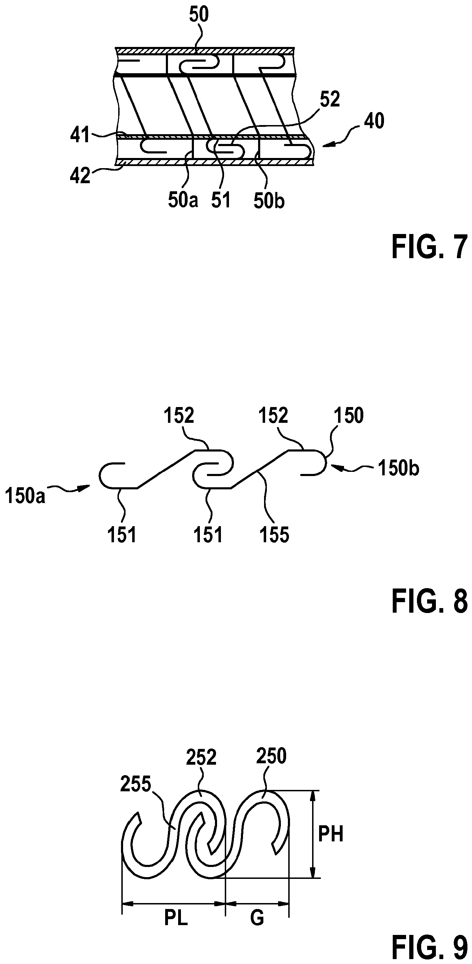

Einen Ausschnitt aus dem proximalen Abschnitt 45 der Versteifungshülse 40, der in Längsrichtung A zwischen dem distalen Abschnitt 46 und dem Konnektor 13 angeordnet ist, ist in Fig. 7 gezeigt. Der proximale Abschnitt 45 wird durch eine erfindungsgemäße röhrenförmige Struktur aus einem S-förmigen Profilband 50 gebildet, das in Form einer Helix angeordnet und in Fig. 5 im Querschnitt gezeigt ist. Das S-förmige Profilband 50 ist entlang der röhrenförmigen Struktur, die die Versteifungshülse 40 bildet, derart helixförmig gewunden, dass es einen Hohlzylinder ausbildet. Das S-förmige Profilband ist mit einer konstanten Steigung entlang des Umfangs U des proximalen Abschnitts 45 der Versteifungshülse 40 gewunden.

Das Profilband 50 besitzt einen S-förmigen Querschnitt, der in Fig. 5 gezeigt ist. Jedes Profilband 50 ist durch Biegung aus einem Flachband mit einer Dicke von beispielsweise d = 0,005 mm bis 0,02 mm und einer Breite von beispielsweise 0,2 mm bis 10 mm hergestellt. Die Länge des Flachbands bzw. Profilbands 50 kann beispielsweise 1000 mm betragen. Die Länge des Profilbands 50 ist abhängig von der Breite des umgeformten Profilbands, dem Durchmesser der Versteifungshülse 40 und der Steigung der Helix (bzw. der Anzahl Windungen). Bezogen auf die Längsrichtung A der Versteifungshülse 40 besitzt jedes Profilband 50 proximal und distal jeweils einen U-förmigen Längsabschnitt 51, 52, welche durch einen mittigen geraden Abschnitt 55 verbunden sind. Der mittige gerade Abschnitt 55 des Querschnitts verläuft entweder in radialer Richtung bezogen auf die Versteifungshülse 40 (siehe Fig. 5 und 7) oder schräg zur radialen Richtung (siehe Fig. 6 und 8). Entsprechend verläuft in dem ersten Ausführungsbeispiel der mittige Abschnitt 55 senkrecht zu den Schenkeln der U-förmigen Längsabschnitte 51, 52 (siehe Fig. 5 und 7) oder unter einem deutlich von 90° verschiedenen Winkel (siehe Abschnitt 155 in Fig. 6 und 8).

Die beiden U-förmigen Längsabschnitte 51, 52 eines Profilbands 50 sind in verschiedene Richtungen gebogen, nämlich der erste, distal angeordnete U-förmige Längsabschnitt 51 in radialer Richtung nach außen und der zweite, proximal angeordnete U-förmige Längsabschnitt 52 nach innen. Die Biegerichtung kann auch jeweils umgekehrt sein. Fig. 7 zeigt das Ineinandergreifen des helixförmig entlang des Umfangs U der Katheterhülse 40 gewundenen Profilbands 50, wobei der Abschnitt 50a zu einem ersten Gang der Helix und der Abschnitt 50b zu einem zweiten Gang der Helix gehört, der zu dem ersten Gang benachbart ist. Das Profil besitzt eine Länge PL sowie eine Höhe PH, wobei die Parameter des Profilbands 50 jeweils in Fig. 5 eingezeichnet sind. Die U-förmigen Längsabschnitte 51, 52 besitzen jeweils gleiche Abmessungen, nämlich einen kürzeren Schenkel mit der Länge a und einen längeren Schenkel mit der Länge PL/2, da die Länge des längeren Schenkels genau der Hälfte der Gesamtlänge PL/2 entspricht. Die Höhe des U-förmigen Längsabschnitts 51, 52 wird jeweils mit h bezeichnet. Die benachbarten Abschnitte 50a, 50b, die benachbarten Gängen der Helix entsprechen, greifen derart ineinander, dass der Schenkel des ersten, nach außen gebogenen U-förmigen Längsabschnitts 51 in dem durch die beiden Schenkel des "U" ausgebildeten Hohlraums des zweiten, nach innen gebogenen U-förmigen Längsabschnitts 52 des ersten Profilbands 50a angeordnet ist. Entsprechend kann das Ineinandergreifen der Profilbänder erfolgen, wenn die Biegerichtung der Profilbänder umgekehrt ist.

Bevorzugte Ausführungsformen des erfindungsgemäßen proximalen Abschnitts 45 der Versteifungshülse 40 weisen die in Tabelle 1 offenbarten Maße auf.

**Tabelle 1**

| | PL | PL/2 | a | d | h | H | Versteifungsschlauch Außendurchmesser | Versteifungsschlauch Innendurchmesser | Versteifungsschlauch Länge | Anz. Wicklung en | Min. Biegeradius |
|---|---|---|---|---|---|---|---|---|---|---|---|
| V1 | 0.2 | 0.1 | 0.06 | 0.01 | 0.08 | 0.1 | 5.9 | 5.7 | 100 | 752 | 36 |
| V2 | 1 | 0.5 | 0.14 | 0.01 | 0.08 | 0.1 | 5.9 | 5.7 | 100 | 150 | 37 |
| V3 | 8 | 4 | 2.55 | 0.01 | 0.08 | 0.1 | 5.9 | 5.7 | 100 | 22 | 42 |

| | | | | | | | | | | | |
|---|---|---|---|---|---|---|---|---|---|---|---|
| *) Alle Dimensionen in mm | | | | | | | | | | | |

Fig. 8 zeigt analog das Ineinandergreifen der benachbarten Gänge des in Fig. 6 dargestellten zweiten Ausführungsbeispiels eines Profilbands 150 mit einem schräg verlaufenden mittigen Abschnitt 155, wobei das Profilband 150 ansonsten ähnlich zu dem in den Fig. 5 und 7 gezeigten Profilband 50 geformt ist.

Durch dieses Ineinandergreifen können die Gänge des Profilbands 50, 150 entsprechenden Abschnitten 50a, 50b, 150a, 150b in Längsrichtung A der Katheterhülse 4 beziehungsweise der Versteifungshülse 40 zueinander verschoben werden. Dies wird insbesondere dadurch begünstigt, dass der Schenkel des U-förmigen Längsabschnitts 51, 52, 151, 152 der mit dem jeweils anderen U-förmigen Längsabschnitt über den mittigen Abschnitt 55,155 verbunden ist, länger ist als der jeweils andere, gegenüber liegende Schenkel. Weiterhin ist es möglich, die nebeneinander liegenden Gänge der Helix des S-förmigen Profilbands 50, 150 zueinander zu verdrehen, so dass sich eine Biegung der Katheterhülse 4 beziehungsweise der Versteifungshülse 40 ergibt. Hieraus resultiert die hohe Biegeflexibilität der erfindungsgemäßen Katheterhülse 4 in ihrem proximalen Abschnitt 45.

Die Figuren 9 bis 15 zeigen weitere Ausführungsbeispiele für eine erfindungsgemäße Struktur.

Das in Fig. 9 gezeigte dritte Ausführungsbeispiel einer erfindungsgemäßen röhrenförmigen Struktur besitzt ein Profilband 250, das gegenüber den oben diskutierten Profilbändern 50 und 150 eine deutlich abgerundetere Form sowie eine verglichen mit der Länge des Profilbands größere Profilhöhe aufweist. Beispielsweise beträgt die Profillänge PL = 0,2 mm, die Ganghöhe der Helix G = 0,133 mm und die Profilhöhe PH = 0,1 mm. Die Katheterhülse bestehend aus der in Fig. 9 gezeigten Struktur kann beispielsweise eine Außendurchmesser OD = 5,9 mm und einen Innendurchmesser ID = 5,7 mm aufweisen. Die Länge der Katheterhülse kann beispielsweise 100 mm betragen. Hierfür wird das Profilband 250 in ca. 750 Helix-Windungen gelegt. Mit einer derartigen Katheterhülse kann beispielsweise ein minimaler Biegeradius von etwa 36 mm realisiert werden.

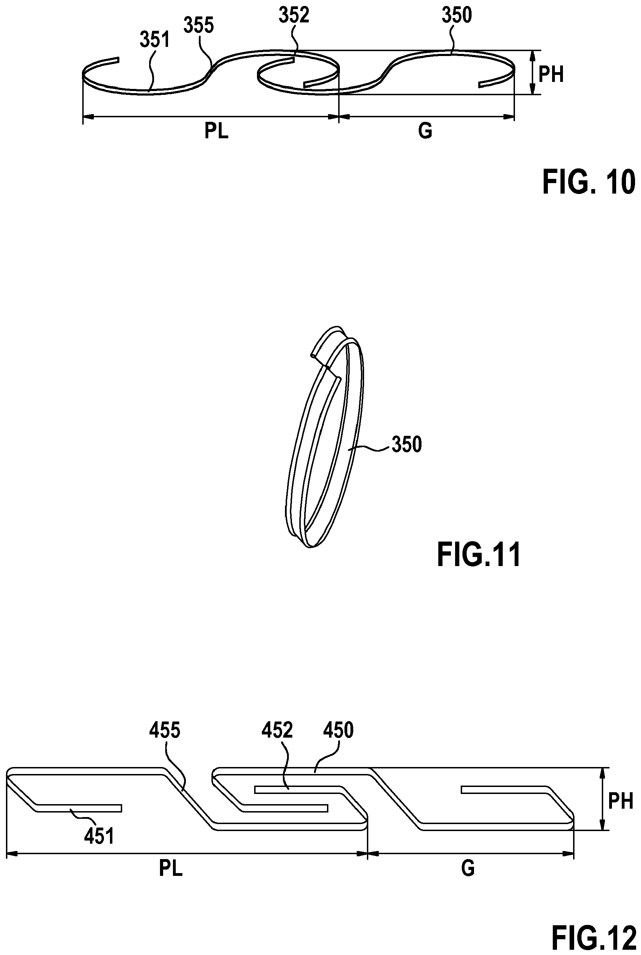

Fig. 10 und 11 veranschaulichen ein viertes Ausführungsbeispiel einer erfindungsgemäßen röhrenförmigen Struktur. Diese ergibt sich aus einem helixförmig gewundenen Profilband 350, das gegenüber dem in Fig. 9 dargestellten Ausführungsbeispiel eine deutlich höhere Profillänge PL = 1 mm verglichen mit der Profilhöhe PH = 0,1 mm aufweist. Daher sind auf einer Länge der Kathetherhülse von 100 mm lediglich ca. 150 Windungen des die Helix ausbildenden Profilbands 350 erforderlich. Mit dieser Struktur wird ein minimaler Biegeradius der aus dieser Struktur bestehenden Katheterhülse von mindestens 37 mm erreicht. Zu diesem Ausführungsbeispiel zeigt Fig. 11 eine Windung (Gang) der Helix aus dem Profilband 350. Weiter ist die Ganghöhe G eingezeichnet.

An den Ausführungsbeispielen der Figuren 9, 10 und 12 zeigt sich der Einfluss der Struktur auf die Eigenschaften der rohrförmigen Struktur. Feinere und dichter ineinander gefügte Profilbänder 250 wie in Figur 9 führen zu einem deutlich kleineren Biegeradius der rohrförmigen Struktur als bei weiteren und weniger dicht ineinander gefügten Profilbändern 450 wie in Figur 12. Die rohrförmige Struktur nach Fig. 9 weist einen Biegeradius von 36 mm auf, die rohrförmige Struktur nach Fig. 10 einen Biegeradius von 37 mm und die rohrförmige Struktur nach Fig. 12 einen Biegeradius von 42 mm. Dafür lassen sich die Profilbänder 450 nach Fig. 12 deutlich leichter ineinander fügen als die Profilbänder 250 nach Fig. 9, so dass die rohrförmige Struktur nach Fig. 12 leichter zu fertigen ist. Der Fachmann wird so entsprechend das Optimum aus Biegeradius und Fertigung der jeweiligen Anwendung wählen.

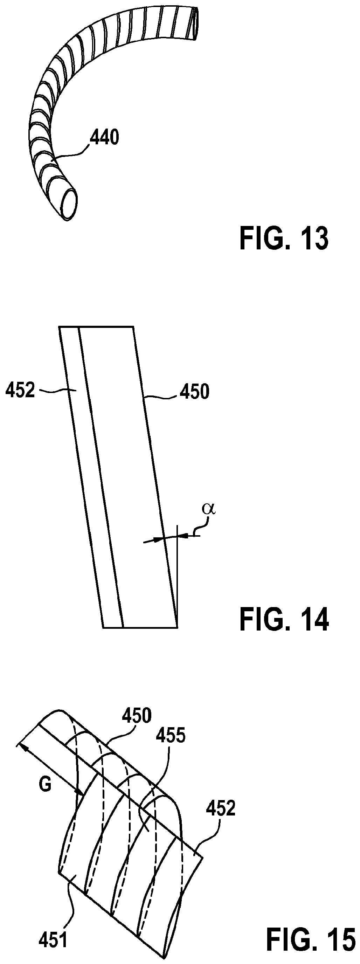

Ein weiteres Ausführungsbeispiel einer röhrenförmigen Struktur ist aus den Fig. 12 bis 15 ersichtlich. Diese Struktur ähnelt dem in Fig. 5 und 8 dargestellten Ausführungsbeispiel, wobei die beiden U-förmigen Längsabschnitte 451 und 452 des Profilbands 450 eine deutlich eckigere Form in einer Querschnittdarstellung verglichen mit dem ersten Ausführungsbeispiel (siehe Fig. 5) aufweisen. Die Profillänge beträgt beispielsweise PL = 8 mm, die Profilhöhe PH = 0,1 mm und die Ganghöhe G = 0,665 mm. Eine Katheterhülse mit einer solchen röhrenförmigen Struktur weist bei einer Gesamtlänge der Katheterhülse von 100 mm ca. 20 Windungen auf und erreicht einen Biegeradius der Katheterhülse von mindestens 42 mm. Eine Versteifungshülse 440 aus einer Struktur nach diesem Ausführungsbeispiel ist in Fig. 13 gezeigt. Ferner veranschaulicht Fig. 15 eine einzige Helix aus dem Profilband 450 mit der Ganghöhe G, welche beispielsweise 4,9 mm beträgt. Fig. 14 zeigt demgegenüber ein abgerolltes Profilband 450 mit einem Steigungswinkel α = 15°.

### Bezugszeichenliste:

- 1: Katheter
- 2a: Handgriff
- 2b: Stabilisierabschnitt
- 3: Außenschaft
- 4: Katheterhülse
- 7: Innenschaft
- 9: Prothesenkonnektor
- 11: röntgenopaker Ring
- 13: proximaler Konnektor
- 40: Versteifungshülse
- 41: erste Polymerlage
- 42: zweite Polymerlage
- 45: proximaler Abschnitt der Versteifungshülse 40
- 46: distaler Abschnitt der Versteifungshülse 40
- 461: Hacken der Krone
- 50, 150, 250, 350, 450: Profilband
- 51, 151, 251, 351, 451: U-förmiger erster Längsabschnitt
- 52, 152, 252, 352, 452: U-förmiger zweiter Längsabschnitt
- 55, 155, 255, 355, 455: mittiger Abschnitt
- 156: Schlitz
- A: Längsrichtung (Längsachse) der Katheterhülse 4
- ID: Innendurchmesser der Katheterhülse 4
- OD: Außendurchmesser der Katheterhülse 4
- U: Umfangsrichtung der Katheterhülse 4
- a: Länge des kürzeren Schenkels des U-förmigen Längsabschnitts 51, 52
- PL/2: Länge des längeren Schenkels des U-förmigen Längsabschnitts 51, 52
- d: Dicke des Profilbands 50
- h: Höhe des U-förmigen Längsabschnitts 51, 52
- PH: Gesamthöhe des Profils des jeweiligen Profilbands im Querschnitt
- PL: Gesamtlänge des Profils des jeweiligen Profilbands im Querschnitt
- G: Ganghöhe der Helix

## Patentansprüche

1. Röhrenförmige Struktur für eine Katheterhülse (4) aufweisend mindestens ein S-förmiges Profilband (50, 150, 250, 350, 450), das entlang der Struktur in einer Helix angeordnet ist, wobei das mindestens eine S-förmige Profilband (50, 150, 250, 350, 450) im Bereich jeder Seitenkante jeweils einen U-förmigen Längsabschnitt (51, 52, 151, 152, 251, 252, 351, 352, 451, 452) aufweist, wobei jeweils ein U-förmiger Längsabschnitt (51, 52, 151, 152, 251, 252, 351, 352, 451, 452) des Profilbands mit dem gegenüber liegenden, U-förmigen Längsabschnitt (51, 52, 151, 152, 251, 252, 351, 352, 451, 452) eines unmittelbar benachbarten Gangs der Helix desselben Profilbands (50, 150, 250, 350, 450) oder eines weiteren, in der Helix benachbart angeordneten S-förmigen Profilbands (50, 150, 250, 350, 450) ineinander greift.

2. Struktur nach Anspruch 1, **dadurch gekennzeichnet, dass** ein mittiger Abschnitt des mindestens einen S-förmigen Profilbands (50, 150, 250, 350, 450) im Querschnitt entweder in radialer Richtung oder schräg zur radialen Richtung verläuft.

3. Struktur nach einem der vorhergehenden Ansprüche, **dadurch gekennzeichnet, dass** die Helix rechtsgängig oder linksgängig ausgebildet ist.

4. Struktur nach einem der vorhergehenden Ansprüche, **dadurch gekennzeichnet, dass** der direkt mit dem mittigen Abschnitt (55, 155, 255, 355, 455) verbundene Schenkel jedes U-förmigen Längsabschnitts (51, 52, 151, 152, 251, 252, 351, 352, 451, 452) jedes S-förmigen Profilbands (50, 150, 250, 350, 450) länger ist als der andere Schenkel.

5. Struktur nach einem der vorhergehenden Ansprüche, **dadurch gekennzeichnet, dass** das S-förmige Profilband vorzugsweise mindestens ein metallisches Material der Gruppe umfassend Stahl, Co-Cr-Legierungen, Nitinol und Kupfer-Legierungen und/oder ein steifes Polymermaterial aufweist.

6. Katheterhülse (4), insbesondere zum Einbringen einer stentgestützten Herzklappenprothese, mit einer Versteifungshülse (40) und einer ersten Polymerlage (41), die in radialer Richtung innerhalb der Versteifungshülse (40) angeordnet ist, und einer zweiten Polymerlage (42), die in radialer Richtung außerhalb der Versteifungshülse (40) angeordnet ist, wobei die Struktur nach einem Ansprüche 1 bis 4 einen mittigen und/oder proximalen Abschnitt (46) der Versteifungshülse (40) ausbildet.

7. Katheterhülse (4), insbesondere zum Einbringen einer stentgestützten Herzklappenprothese, mit einer Versteifungshülse (40) und einer ersten Polymerlage (41), die in radialer Richtung innerhalb der Versteifungshülse (40) angeordnet ist, und einer zweiten Polymerlage (42), die in radialer Richtung außerhalb der Versteifungshülse (40) angeordnet ist, wobei die Struktur nach einem Ansprüche 1 bis 4 über die gesamte Länge der Versteifungshülse (40) ausbildet

8. Katheter (1) mit einer Katheterhülse (4) nach Anspruch 6 oder 7, wobei die Katheterhülse (4) mit einem Außenschaft (3) des Katheters (1) verbunden ist und zur Aufnahme eines Implantats ausgebildet ist.

9. Katheter (1) nach Anspruch 8, wobei die Versteifungshülse (40) über einen metallischen Verbinder mit dem Außenschaft (3) verbunden ist.

10. Katheter nach Anspruch 8 oder 9, wobei die erste Polymerlage (41) , bevorzugt nahtlos, in eine innere Polymerlage des Außenschaftes (3) und die zweite Polymerlage (42), bevorzugt nahtlos, in eine äußere Polymerlage des Außenschaftes (3) übergeht, wobei besonders bevorzugt der Außenschaft zwischen innerer und äußerer Polymerlage eine metallische Verstärkung aufweist.

11. Katheter nach einem der Ansprüche 7 bis 10, wobei die Versteifungshülse (40) mit einer distalen Krone (46) verbunden ist.

12. Katheter nach Anspruch 11, wobei die Verbindung über mindestens ein U-Profil erfolgt, welches in das Profil der rohrförmigen Struktur greift.

13. Katheter nach Anspruch 11, wobei die Verbindung über mindestens ein Haken 461 erfolgt, welcher in ein Schlitz 156 der rohrförmigen Struktur greift.
